# EUROPÄISCHE PATENTSCHRIFT

(11) **EP 3 180 211 B1**
(45) Veröffentlichungstag und Bekanntmachung des Hinweises auf die Patenterteilung: **29.08.2018**
(21) Anmeldenummer: 15732570.5
(22) Anmeldetag: 18.06.2015
(51) Int. Cl.: B60R 11/04, A61B 5/18, H04N 5/225, B60K 37/02

(54) **FAHRERBEOBACHTUNGSSYSTEM IN EINEM KRAFTFAHRZEUG**
DRIVER OBSERVATION SYSTEM IN A MOTOR VEHICLE
SYSTÈME DE SURVEILLANCE DU COMPORTEMENT DE CONDUITE POUR VÉHICULE À MOTEUR

(30) Priorität: 11.08.2014 DE 102014215856
(43) Veröffentlichungstag der Anmeldung: 21.06.2017
(73) Patentinhaber: Robert Bosch GmbH, 70442 Stuttgart (DE)
(72) Erfinder: TRINH, Hoang, 70839 Gerlingen (DE); LINDNER, Markus, 70180 Stuttgart (DE)
(86) Internationale Anmeldenummer: PCT/EP2015/063735
(87) Internationale Veröffentlichungsnummer: WO 2016/023662

(56) Entgegenhaltungen:
- WO-A1-2014/017061
- WO-A1-2014/103141
- US-A1- 2003 201 895
- US-A1- 2004 047 058
- US-A1- 2008 068 462
- US-A1- 2011 025 836

## Beschreibung

Die Erfindung betrifft ein Fahrerbeobachtungssystem in einem Kraftfahrzeug, mit wenigstens einer Kameraeinrichtung, die wenigstens eine auf einem Trägerelement angeordnete Kameraeinheit und wenigstens einen Infrarotstrahler aufweist.

### Stand der Technik

Fahrerbeobachtungssysteme der Eingangs genannten Art sind aus dem Stand der Technik bekannt. Um das Fahrerverhalten zu erfassen, beispielsweise um feststellen zu können, ob der Fahrer das Verkehrsgeschehen beobachtet oder Müdigkeitserscheinungen aufweist, sind kamerabasierte Fahrerbeobachtungssysteme bekannt, die das Gesicht des Fahrers erfassen und in Abhängigkeit der erfassten Gesichtszüge oder der Ausrichtung des Fahrerkopfes auf das Verhalten des Fahrers schließen. Um derartige Beobachtungen auch dann noch durchführen zu können, wenn die Fahrgastzelle dunkel ist, beispielsweise während einer Nachtfahrt, ist bekannt, einen Infrarotstrahler vorzusehen, der den Fahrer anstrahlt, so dass eine Kameraeinheit der Kameraeinrichtung, insbesondere eine Infrarotkamera, den Fahrer auch im Dunkeln erfassen kann.

Die Kameraeinheit, die in der Regel aus einem Kamerachip und einer dem Chip zugeordneten Optik zum gezielten Leiten des Lichts besteht, sowie der Infrarotstrahler müssen vorteilhaft in dem Kraftfahrzeug angebracht werden können, um den Fahrer sicher beobachten zu können. Die Integration der Kameraeinrichtung in das Kraftfahrzeug spielt somit eine wesentliche Rolle bei der Effektivität des Systems.

Die Offenlegungsschrift WO 2014/017061 A1 offenbart, gemäß dem Oberbegriff des Anspruchs 1, ein Fahrerbeobachtungssystem für ein Fahrzeug mit einer Kameraeinrichtung, die zumindest einen Infrarotstrahler und eine Kameraeinheit aufweist, die gemeinsam auf einer Leiterplatte angeordnet sind. Die Kameraeinrichtung ist dabei auf einer Lenksäule eines Lenkrades angeordnet und derart ausgerichtet, dass das Gesicht eines Fahrers erfasst wird. Die US 2008/068462 A1 zeigt ein Fahrerbeobachtungssystem für ein Fahrzeug mit einer Kameraeinheit, die zur Erfassung von Licht im Infrarotbereich ausgebildet ist, wobei ein Fahrer des Fahrzeugs mit infrarotem Licht beleuchtet wird. Die Kameraeinheit ist auf einer Leiterplatte hinter einem für infrarotes Licht transparenten Bereich einer Instrumentenanzeige eines Kombiinstruments angeordnet.

### Offenbarung der Erfindung

Das erfindungsgemäße Fahrerbeobachtungssystem mit den Merkmalen des Anspruchs 1 hat den Vorteil, dass die Kameraeinrichtung besonders kompakt und damit modulartig in dem Kraftfahrzeug verbaubar ist, wobei die Kameraeinheit und der Infrarotstrahler zusammen als eine handhabbare Einheit vorliegen, so dass auch eine elektrische Kontaktierung von Kamera und Infrarotstrahler einfach und kostengünstig gestaltbar ist. Erfindungsgemäß ist hierzu vorgesehen, dass das Trägerelement, auf welchem die Kameraeinheit üblicherweise angeordnet ist, zusätzlich eine Einrichtung zur Arretierung des Infrarotstrahlers an dem Trägerelement aufweist. Somit wird der Infrarotstrahler am gleichen Trägerelement befestigt, an welchem auch die Kameraeinheit gehalten ist. Dadurch wird eine kompakte Einheit geboten, die auf einfache Art und Weise in dem Kraftfahrzeug verbaut werden kann. Durch das gemeinsame Trägerelement ist auch eine elektrische Kontaktierung von Kamera und Infrarotstrahler leicht realisierbar. So kann beispielsweise das Trägerelement mit einem einzigen Kontaktstecker oder -Anschluss versehen sein, mit welchem sowohl die Kamera als auch der Infrarotstrahler elektrisch kontaktierbar sind. Durch die räumliche Nähe von Kamera und Infrarotstrahler wird außerdem gewährleistet, dass eine optimale Fahrerausleuchtung und -beobachtung erfolgt. Gemäß einer vorteilhaften Weiterbildung der Erfindung ist vorgesehen, dass die Einrichtung zu lösbaren Arretierung des Infrarotstrahlers ausgebildet ist.

Während es grundsätzlich denkbar ist, die Einrichtung beispielsweise in Art einer Löt- oder Schweißstelle auszubilden, um eine dauerhafte Arretierung des Infrarotstrahlers an dem Trägerelement vorzusehen, ist es gemäß der bevorzugten Ausführungsform vorgesehen, dass der Infrarotstrahler lösbar an dem Trägerelement gehalten ist. Dadurch ergeben sich mehrere Vorteile. Zum einen lässt sich der Infrarotstrahler auf einfache Art und Weise austauschen, beispielsweise, wenn ein Defekt vorliegt. Zum anderen ist es dadurch möglich, die Kameraeinrichtung modular zu gestalten, so dass beispielsweise in bestimmten Anwendungsfällen die Kameraeinrichtung ohne den Infrarotstrahler und in anderen Anwendungsfällen zusammen mit zumindest einem Infrarotstrahler verbaut werden kann.

Besonders bevorzugt ist vorgesehen, dass das Trägerelement wenigstens zwei Einrichtungen zur lösbaren Arretierung jeweils eines Infrarotstrahlers aufweist. Die Einrichtungen sind dabei zweckmäßigerweise identisch zueinander ausgebildet, so dass ein Infrarotstrahler sowohl durch die eine als auch durch die andere Einrichtung an dem Trägerelement lösbar arretiert werden kann. Dadurch wird die Modularität der Kameraeinrichtung weiter gesteigert, da bei der Montage entschieden werden kann, ob ein oder zwei Infrarotstrahler verwendet werden sollen. Selbstverständlich können auch mehr als nur zwei Einrichtungen zur lösbaren Arretierung an dem Trägerelement vorgesehen sein. Vorzugsweise sind die Einrichtungen nebeneinander angeordnet. Alternativ ist es auch denkbar, eine Einrichtung auf einer Seite und die andere Einrichtung auf der anderen Seite der Kamera auf dem Trägerelement vorzusehen, um beispielsweise den Fahrer von zwei Seiten auszuleuchten.

Gemäß einer vorteilhaften Weiterbildung der Erfindung ist außerdem vorgesehen, dass die Einrichtung oder die Einrichtungen jeweils als Steckaufnahme beziehungsweise Steckaufnahmen ausgebildet sind. Der Infrarotstrahler lässt sich somit einfach in die jeweilige Steckaufnahme zu seiner Arretierung einsetzen. Durch die Ausbildung als Steckaufnahme wird die Montage besonders einfach gestaltet.

Bevorzugt ist vorgesehen, dass die jeweilige Einrichtung Rastmittel zum Arretieren des einen oder der mehreren Infrarotstrahler aufweist. Bei den Rastmitteln kann es sich beispielsweise um elastisch verformbare oder bewegbare Rastnasen handeln, die mit entsprechenden Rastaussparungen oder Rasthintergriffsitzen des oder der Infrarotstrahler zusammenwirken.

Erfindungsgemäß ist vorgesehen, dass die Kameraeinrichtung in ein Kombiinstrument eines Kraftfahrzeugs integrierbar/integriert ist. Das Kombiinstrument liegt in der Regel stets im Blickfeld des Fahrers und wird allenfalls vom Lenkrad oder von einem Arm des Fahrers beim Lenken bereichsweise verdeckt. Durch Vorsehen mehrerer entsprechender Kameraeinrichtungen in dem Kombiinstrument kann erreicht werden, dass der Fahrer durchgängig beobachtet werden kann. Darüber hinaus stehen im Kombiinstrument bereits ausreichend Möglichkeiten zum elektrischen Kontaktieren der Kameraeinrichtung zur Verfügung, so dass die Integration der Kameraeinrichtung in das Kombiinstrument leicht realisierbar ist. Vorzugsweise ist die wenigstens eine Kameraeinrichtung seitlich oder mittig in dem Kombiinstrument angeordnet. Sind mehrere Kameraeinrichtungen vorgesehen, so sind diese vorzugsweise an den Seitenrändern des Kombiinstruments angeordnet, um die Wahrscheinlichkeit der durchgehenden Beobachtung des Fahrers zu maximieren.

Ferner ist bevorzugt vorgesehen, dass das Trägerelement als Leiterplatte ausgebildet ist. Damit ist die elektrische Kontaktierung von Kamera und Infrarotstrahler besonders einfach. Vorzugsweise weist die Leiterplatte mehrere Leiterbahnen auf, die zu der den Infrarotstrahler arretierenden Einrichtung und zu der Kameraeinheit führen. Gegebenenfalls sind die Rastmittel derart ausgebildet, dass durch diese eine Vorspannung erzeugt wird, durch welche der Infrarotstrahler mit seinen Kontaktanschlüssen gegen eine Leiterbahn der Leiterplatte beziehungsweise eine entsprechende Kontaktstelle der Leiterplatte gedrängt wird, um einen dauerhaften elektrischen Berührungskontakt zu gewährleisten. Auf der Leiterplatte kann außerdem auch eine Steuereinheit zum Betreiben der Kameraeinrichtung angeordnet sein. Zumindest aber weist die Leiterplatte vorzugsweise einen Kontaktanschluss für das Kombiinstrument auf, um eine einfache Anschlussmöglichkeit und Integration des Kombiinstrumentes zu gewährleisten.

Erfindungsgemäß sind Mittel vorgesehen, durch welche die Kameraeinrichtung in das Kombiinstrument, insbesondere in eine Leiterplatte des Kombiinstruments einsteckbar, insbesondere einclipsbar ist. Dadurch wird eine besonders einfache Montage der Kameraeinrichtung gewährleistet, die den Montageaufwand für das Fahrerbeobachtungssystem minimiert.

Erfindungsgemäß weist die Leiterplatte des Kombiinstruments wenigstens eine Aussparung zur Aufnahme der Kameraeinrichtung und/oder des Infrarotstrahlers auf. Insbesondere lässt sich dadurch ein Verclipsen der Kameraeinrichtung direkt in der Leiterplatte des Kombiinstruments realisieren.

Ferner ist bevorzugt vorgesehen, dass der Infrarotstrahler wenigstens eine Infrarot-Leuchtdiode (Infrarot-LED) aufweist. Die Infrarot-Leuchtdiode weist eine hohe Lebensdauer auf und ist kostengünstig in das Fahrerbeobachtungssystem integrierbar. Insbesondere ist vorgesehen, dass die Infrarot-Leuchtdiode direkt in die Steckaufnahme der Einrichtung einsteckbar und darin arretierbar ist. Gegebenenfalls kann auch vorgesehen sein, dass die Steckaufnahme zur Aufnahme mehrerer Infrarot-Leuchtdioden ausgebildet ist.

Gemäß einer alternativen Ausführungsform ist bevorzugt vorgesehen, dass auf dem Trägerelement, insbesondere auf dem in das Kombiinstrument einsetzbaren Trägerelement der Kameraeinrichtung nur die Kameraeinheit vorgesehen ist, während der eine oder mehrere Infrarotstrahler unabhängig von der Kameraeinrichtung in das Kombiinstrument integriert sind, beispielsweise durch Anordnen auf einer Leiterplatte des Kombiinstruments. Außerdem ist es denkbar, zusätzlich zu den Infrarotstrahlern der Kameraeinrichtung weitere Infrarotstrahler außerhalb des Kombiinstruments, insbesondere im Bereich des Armaturenbretts des Kraftfahrzeugs vorzusehen.

Im Folgenden soll die Erfindung anhand eines Ausführungsbeispiels näher erläutert werden. Dazu zeigen:
- Figur 1: ein Fahrerbeobachtungssystem in einem Kraftfahrzeugs,
- Figur 2: eine Seitenansicht des Fahrerbeobachtungssystems,
- Figur 3: eine Draufsicht auf das Fahrerbeobachtungssystems,
- Figur 4: ein weiteres Ausführungsbeispiel des Fahrerbeobachtungssystems,
- Figur 5: ein weiteres Ausführungsbeispiel des Fahrerbeobachtungssystems und
- Figur 6: ein weiteres Ausführungsbeispiel des Fahrerbeobachtungssystems.

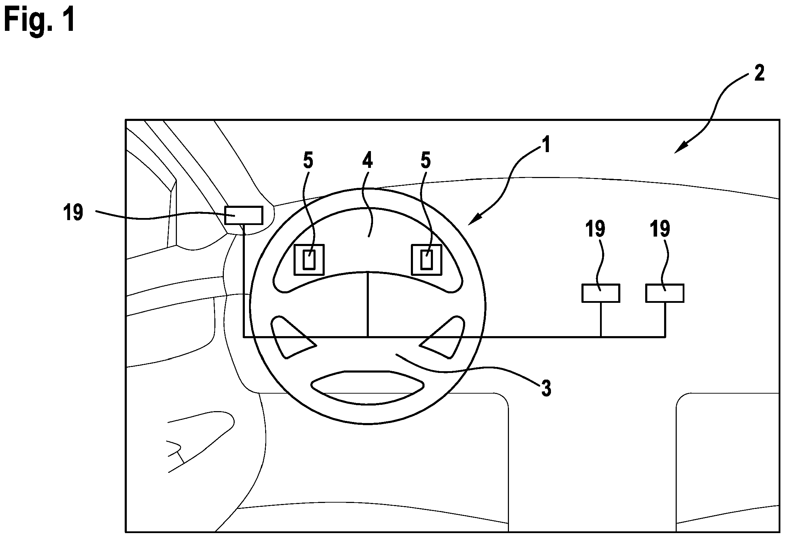

Figur 1 zeigt ein Fahrerbeobachtungssystem 1 für ein hier nur angedeutetes Kraftfahrzeug 2. Das Kraftfahrzeug 2 weist auf der Fahrerseite am Armaturenbrett ein Lenkrad 3 und dahinter, in das Armaturenbrett integriert, ein Kombiinstrument 4 auf. Das Kombiinstrument 4 dient zur Darstellung von aktuellen Zustandswerten des Kraftfahrzeugs, wie beispielsweise der aktuellen Geschwindigkeit, einer Drehzahl einer Antriebseinrichtung oder weiterer Informationen. Die Anzeigen sind dabei insbesondere auf einer Leiterplatte 6 des Kombiinstruments 4 angeordnet. Das Fahrerbeobachtungssystem 1 ist vorliegend in das Armaturenbrett und insbesondere in das Kombiinstrument 4 integriert. Dazu sind in dem Kombiinstrument 4 zwei Kameraeinrichtungen 5 vorgesehen, die an den seitlichen Randbereichen des Kombiinstruments 4 liegen.

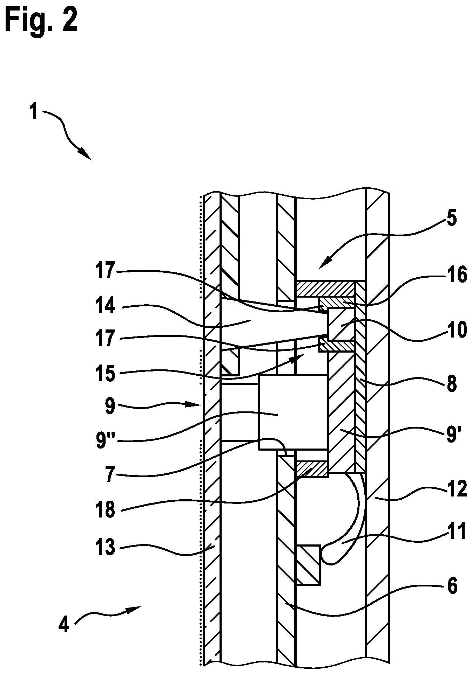

Figur 2 zeigt eine Detailansicht einer der Kameraeinrichtungen 5 des Fahrerbeobachtungssystems 1. Figur 2 zeigt dabei eine Schnittdarstellung durch das Kombiinstrument 4, das die zuvor bereits genannte Leiterplatte 6 aufweist. Die Leiterplatte 6 weist eine Aussparung 7 auf, in welcher die Kameraeinrichtung 5 eingesetzt ist.

Die Kameraeinrichtung 5 weist ein Trägerelement 8 auf, an welchem eine Kameraeinheit 9 mit einem Kamerachip 9' und einer Optik 9", wie beispielsweise eine optische Linse, sowie ein Infrarotstrahler 10 angeordnet sind. Das Trägerelement 8 ist als Leiterplatte ausgebildet, die den Infrarot--Strahler und die Kameraeinheit 9 elektrisch kontaktiert. Von der Leiterplatte beziehungsweise dem Trägerelement 8 geht außerdem ein Flachbandkabel 11 ab, das zu der Leiterplatte 6 des Kombiinstruments 4 führt und dadurch die Kameraeinrichtung 5 mit dem Kombiinstrument 4 elektrisch verbindet. Das Trägerelement 8 liegt dabei rückseitig an einer Gehäusewand 12 des Kombiinstruments an und ist dort beispielsweise verclipst, verklebt oder verschraubt. Auf der der Gehäusewand 12 gegenüberliegenden Seite wird das Kombiinstrument 4 und damit auch die Kameraeinrichtung 5 durch ein Abdeckglas 13, das insbesondere eine Anti-Reflektionsbeschichtung aufweist, überdeckt. Im Bereich des Infrarot-Strahlers 10 sowie der Kameraeinheit 9 ist die Anti-Reflektionsbeschichtung vorzugsweise unterbrochen.

Eine Optik 9" der Kameraeinheit 9 der Kameraeinrichtung 5 erstreckt sich durch die Aussparung 7 hindurch bis an das Abdeckglas 13. Ebenso erstreckt sich ein Lichtschacht 14 des Infrarotstrahlers bis an das Abdeckglas 13 heran.

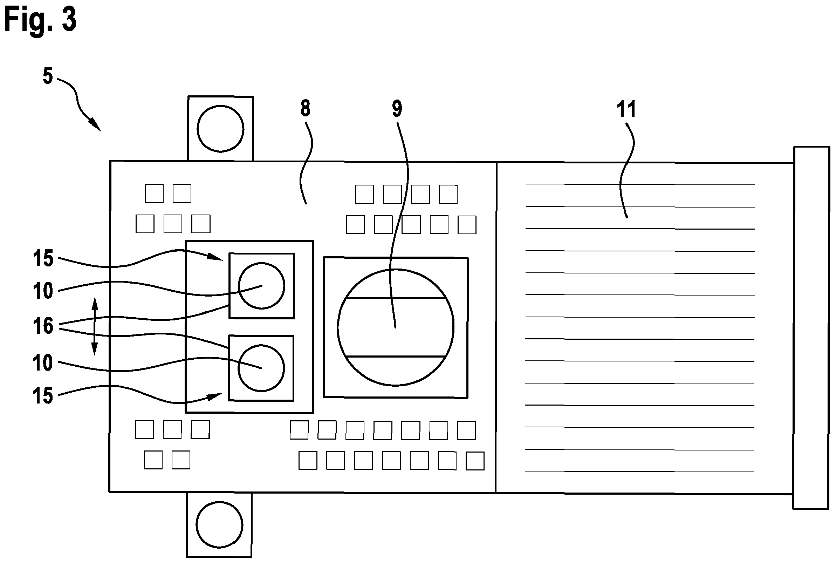

Figur 3 zeigt eine Draufsicht auf die Kameraeinrichtung 5. Auf dem Trägerelement 8 sind die Kameraeinheit 9 sowie zwei der Infrarotstrahler 10 angeordnet. Dabei sind die Infrarotstrahler 10 nebeneinanderliegend und beabstandet zu der Kameraeinheit 9 vorgesehen. Das Trägerelement 8 weist für jeden der Infrarotstrahler 10 jeweils eine Einrichtung 15 zu dessen Arretierung an dem Trägerelement 8 auf. Die Einrichtungen 15 sind dabei als Steckaufnahmen 16 ausgebildet, in welche der jeweilige Infrarotstrahler 10 zu seiner Arretierung einsteckbar ist. Vorzugsweise weisen die Steckaufnahmen 16 dazu Rastnasen 17 auf, wie sie in Figur 2 angedeutet sind, um eine lösbare Arretierung für den jeweiligen Infrarotstrahler 10 zur Verfügung zu stellen. Optional kann vorgesehen sein, dass auch für die Kameraeinheit 9 auf dem Trägerelement 8 eine Einrichtung zur lösbaren Arretierung der Kameraeinheit 9 vorgesehen ist. Durch die Ausbildung der Einrichtungen 15 als Steckaufnahmen 16 wird auch die elektrische Kontaktierung der Infrarotstrahler 10 bei der Montage automatisch hergestellt.
Durch die Ausbildung der Einrichtungen 15 zur lösbaren Arretierung der Infrarotstrahler 10 ist es möglich, die Anzahl der genutzten Infrarotstrahler modular zu gestalten beziehungsweise zu variieren. Insbesondere ist es dadurch möglich, weniger als die maximale Anzahl möglicher Infrarotstrahler 10 an der Kameraeinrichtung 5 vorzusehen. In Abhängigkeit von der Anzahl der Einrichtungen 15 beziehungsweise der Steckaufnahme 16 können entsprechend viele Infrarotstrahler 10 an dem Trägerelement 8 angeordnet werden. Vorzugsweise weist der Infrarotstrahler 10 eine oder mehrere Infrarot-Leuchtdioden auf, die besonders bevorzugt unabhängig voneinander ansteuerbar sind.
Vorzugsweise sind, wie in Figur 2 gezeigt, der jeweiligen Kameraeinrichtung 5 Rastmittel 18 zugeordnet, die an der Leiterplatte 6 des Kombiinstruments 4 vorgesehen sind, und zur lösbaren Arretierung der jeweiligen Kameraeinrichtung 5 dienen. In Figur 2 sind die Rastmittel vereinfacht dargestellt. Für die Ausbildung des Fahrerbeobachtungssystems 1 gemäß Figur 1 sind entsprechend zwei Aussparungen 7 in der Leiterplatte 6 des Kombiinstruments 4 an den entsprechenden Stellen notwendig. Zusätzliche Infrarotstrahler 19 können, wie in Figur 1 angedeutet, auch außerhalb des Kombiinstruments 4 in das Armaturenbrett des Kraftfahrzeugs 2 integriert werden. Die Infrarotstrahler 10, 19 und die Kameraeinheiten 9 der Kameraeinrichtungen 5 sind dabei stets auf die Position des Fahrers beziehungsweise dessen Kopfes ausgerichtet. Die Infrarotstrahler senden Infrarotlicht aus, das an dem Fahrer reflektiert und durch die Kameraeinheiten 9 erfasst wird. Dadurch ist es möglich, auch bei dunkeln Umgebungsverhältnissen den Fahrer zu beobachten, ohne dass dieser davon gestört wird. Durch das Fahrerbeobachtungssystem 1 kann dadurch beispielsweise eine Gesichtserkennung, eine Kopfverfolgung, eine Augenbeobachtung zur Feststellung von Müdigkeit oder dergleichen durchgeführt werden.

Durch die Ausbildung der jeweiligen Kameraeinrichtung 5 als ein in das Kombiinstrument 4 integrierbares Kameramodul, wie oben stehend beschrieben, ist die Montage des Fahrerbeobachtungssystems 1 besonders einfach. Darüber hinaus wird die Variabilität des Fahrerbeobachtungssystems 1 durch die Einrichtungen 15 erhöht, so dass das Kameramodul in unterschiedlichen Ausführungsformen in das Kombiinstrument 4 beziehungsweise ein entsprechendes Kombiinstrument einsetzbar ist, wobei beispielsweise die Anzahl der Infrarotstrahler 10 in Abhängigkeit von dem jeweiligen Anwendungsfall, beispielsweise in Abhängigkeit vom jeweiligen Kraftfahrzeugtyp, verändert wird. Ebenso ist es denkbar, das Kameramodul beziehungsweise die jeweilige Kameraeinrichtung 5 an einer anderen Stelle, beispielsweise für eine Umfeldsensorik, zu verwenden. Insbesondere ist es denkbar, die Kameraeinrichtung 5 auch ohne Infrarotstrahler 10 für eine Umfeldsensorik zu nutzen. Es ergibt sich somit ein universell einsetzbare Kameraeinrichtung 5, insbesondere für den Kraftfahrzeugbau.

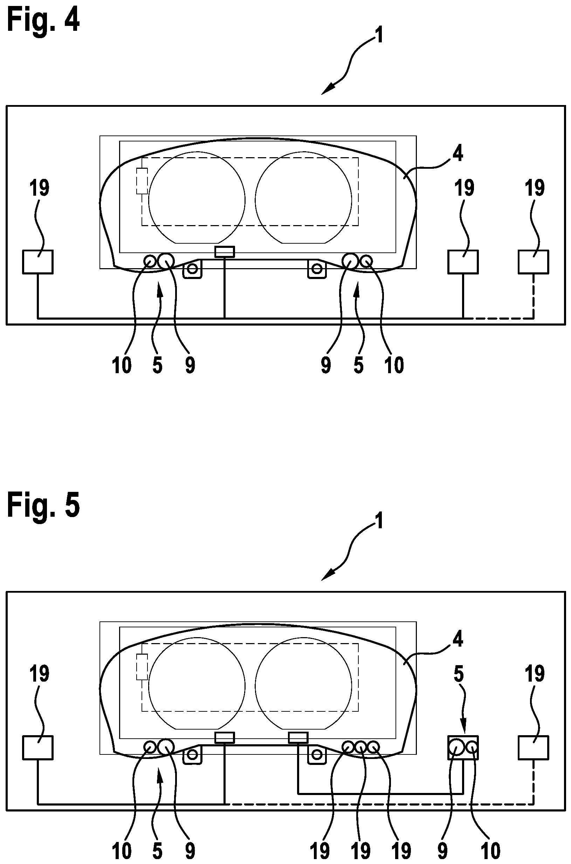

Figuren 4 und 5 zeigen beispielhaft unterschiedliche Anordnungen des Fahrerbeobachtungssystems 1, wobei gemäß Figur 4 zwei Kameraeinrichtungen 5 im unteren Bereich eines Kombiinstruments 4 vorgesehen sind. Figur 5 zeigt ein alternatives Ausführungsbeispiel, bei welchem nur eine Kameraeinrichtung 5 in das Kombiinstrument 4 integriert ist, während eine zweite Kameraeinrichtung 5 in das Armaturenbrett beabstandet zu dem Kombiinstrument 4 eingesetzt ist. Es sind somit unterschiedliche Konfigurationen des Fahrerbeobachtungssystems 1 möglich. Durch die Ausbildung der Kameraeinrichtung 5 in Form eines variablen Moduls, lässt sich dieses vielfach anwenden und aufgrund seiner Variabilität anpassen.

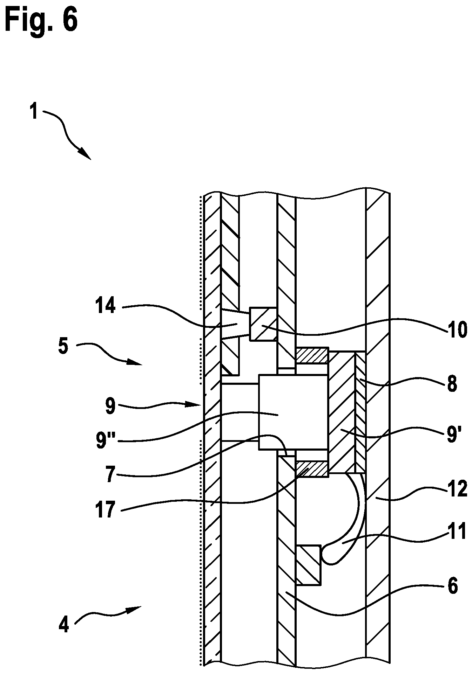

Figur 6 zeigt eine alternative Ausführungsform des Fahrerbeobachtungssystems 1, bei welcher das Trägerelement 8 der Kameraeinrichtung 5 nur zur Aufnahme der Kamera 9, nicht jedoch zur Aufnahme des Infrarotstrahlers 10 dient. In diesem Fall ist also der Infrarotstrahler 10 losgelöst von der Kamera 9 anordenbar. Insbesondere ist gemäß der vorliegenden Ausführungsform vorgesehen, dass der Infrarotstrahler 14 direkt auf der Leiterplatte 6 des Kombiinstruments 4 angeordnet ist. Durch die Verbindung des Flachbandkabels 11 wird die Kamera 9 mit dem Kombiinstrument 4 elektrisch verbunden, wobei der Infrarotstrahler 10 direkt durch die Leiterplatte 6 beispielsweise an einem auf der Leiterplatte 6 angeordneten Mikroprozessor oder dergleichen verbunden ist, der die von der Kamera 9 mithilfe des Infrarotstrahlers 10 erfassten Daten auswertet. In diesem Fall besteht die Kameraeinrichtung 5 also aus zwei Modulbestandteilen, die unabhängig voneinander in das Kombiinstrument 4 oder das Armaturenbrett des Kraftfahrzeugs 2 einbringbar sind. Zumindest dem Trägerelement 8 und der Kameraeinheit 9 sind dabei die zuvor beschriebenen Rastmittel 18 zugeordnet. Der Infrarotstrahler 10 ist in diesem Fall, nicht gemäß der Erfindung, direkt auf die Leiterplatte 6 aufgelötet oder dort durch eine entsprechende Steckaufnahme, wie sie zuvor beschrieben wurde, lösbar arretiert. Selbstverständlich können auf diese Art und Weise auch mehrere Infrarotstrahler 10 an der Leiterplatte 6 befestigt werden. Weist die Leiterplatte 10 entsprechend viele vorbereitete Steckaufnahmen 16 auf, so ist auch die Positionierung und Anzahl der gewünschten Infrarotstrahler 10 variabel.
Selbstverständlich ist es auch denkbar, bei den oben beschriebenen Ausführungsbeispielen auf die zusätzliche, dem jeweiligen Infrarotstrahler 10 zugeordneter Optik, wie beispielsweise den Lichtschacht 14, zu verzichten, oder eine entsprechend andere Optik vorzusehen. Das Flachbandkabel 11 ist vorzugsweise über Steckelemente einerseits mit dem Trägerelement 8 und dadurch mit den darauf befindlichen Komponenten, und andererseits mit der Leiterplatte 6 verbunden.

## Patentansprüche

1. Fahrerbeobachtungssystem (1) für ein Kraftfahrzeug (2), mit wenigstens einer Kameraeinrichtung (5), die wenigstens eine auf einem Trägerelement (8) angeordnete Kameraeinheit (9) und wenigstens einen Infrarotstrahler (10) aufweist, und wobei das Trägerelement (8) wenigstens eine Einrichtung (15) zur Arretierung des Infrarotstrahlers (10) an dem Trägerelement (8) aufweist und wobei die Kameraeinrichtung (5) in ein Kombiinstrument (4) des Kraftfahrzeugs integrierbar/integriert ist, **gekennzeichnet durch** Mittel, durch welche die Kameraeinrichtung (5) in eine Leiterplatte (6) des Kombiinstruments (4) einclipsbar ist, wobei die Leiterplatte (6) des Kombiinstruments (4) wenigstens eine Aussparung (7) zur Aufnahme der Kameraeinrichtung (5) aufweist.

2. Fahrbeobachtungssystem nach Anspruch 1, **dadurch gekennzeichnet, dass** die Einrichtung (15) zur lösbaren Arretierung des Infrarotstrahlers (10) ausgebildet ist.

3. Fahrerbeobachtungssystem nach einem der vorhergehenden Ansprüche, **dadurch gekennzeichnet, dass** das Trägerelement (8) wenigstens zwei Einrichtungen (15) zur lösbaren Arretierung jeweils eines Infrarotstrahlers (10) aufweist.

4. Fahrerbeobachtungssystem nach einem der vorhergehenden Ansprüche, **dadurch gekennzeichnet, dass** die jeweilige Einrichtung (15) als Steckaufnahme (16) ausgebildet ist.

5. Fahrerbeobachtungssystem nach einem der vorhergehenden Ansprüche, **dadurch gekennzeichnet, dass** die jeweilige Einrichtung (15) Rastmittel (18) zum Arretieren des oder der Infrarotstrahler (10) aufweist.

6. Fahrerbeobachtungssystem nach einem der vorhergehenden Ansprüche, **dadurch gekennzeichnet, dass** das Trägerelement (8) als Leiterplatte ausgebildet ist.

7. Fahrerbeobachtungssystem nach einem der vorhergehenden Ansprüche, **dadurch gekennzeichnet, dass** der Infrarotstrahler (10) wenigstens eine Infrarot-Leuchtdiode aufweist.

## Claims

1. Driver observation system (1) for a motor vehicle (2), comprising at least one camera device (5) which has at least one camera unit (9) arranged on a carrier element (8) and at least one infrared emitter (10), and the carrier element (8) having at least one device (15) for locking the infrared emitter (10) to the carrier element (8), and the camera device being integrated/capable of being integrated into an instrument cluster (4) of the motor vehicle, **characterized by** means by which the camera device (5) can be clipped into a circuit board (6) of the instrument cluster (4), the circuit board (6) of the instrument cluster (4) having at least one cut-out (7) for receiving the camera device (5).

2. Driver observation system according to Claim 1, **characterized in that** the device (15) is configured for the detachable locking of the infrared emitter (10).

3. Driver observation system according to one of the preceding claims, **characterized in that** the carrier element (8) has at least two devices (15) for the detachable locking of respectively one infrared emitter (10).

4. Driver observation system according to one of the preceding claims, **characterized in that** the respective device (15) is configured as a plug-in receptacle (16).

5. Driver observation system according to one of the preceding claims, **characterized in that** the respective device (15) has latching means (18) for locking the infrared emitter or emitters (10).

6. Driver observation system according to one of the preceding claims, **characterized in that** the carrier element (8) is configured as a circuit board.

7. Driver observation system according to one of the preceding claims, **characterized in that** the infrared emitter (10) has at least one infrared light-emitting diode.

## Revendications

1. Système d'observation de conducteur (1) pour un véhicule automobile (2), comprenant au moins un dispositif à caméra (5) qui possède au moins une unité à caméra (9) disposée sur un élément porteur (8) et au moins un projecteur d'infrarouges (10), et l'élément porteur (8) possédant au moins un dispositif (15) destiné à bloquer le projecteur d'infrarouges (10) sur l'élément porteur (8) et le dispositif à caméra (5) pouvant être / étant intégré dans un instrument combiné (4) du véhicule automobile, **caractérisé par** des moyens par le biais desquels le dispositif à caméra (5) peut être enclipsé dans un circuit imprimé (6) de l'instrument combiné (4), le circuit imprimé (6) de l'instrument combiné (4) possédant au moins une cavité (7) destinée à accueillir le dispositif à caméra (5).

2. Système d'observation de conducteur selon la revendication 1, **caractérisé en ce que** le dispositif (15) est configuré pour un blocage libérable du projecteur d'infrarouges (10).

3. Système d'observation de conducteur selon l'une des revendications précédentes, **caractérisé en ce que** l'élément porteur (8) possède au moins deux dispositifs (15) servant au blocage libérable respectivement d'un projecteur d'infrarouges (10).

4. Système d'observation de conducteur selon l'une des revendications précédentes, **caractérisé en ce que** le dispositif (15) respectif est réalisé sous la forme d'un logement d'enfichage (16).

5. Système d'observation de conducteur selon l'une des revendications précédentes, **caractérisé en ce que** le dispositif (15) respectif possède des moyens d'encliquetage (18) pour le blocage du ou des projecteurs d'infrarouges (10).

6. Système d'observation de conducteur selon l'une des revendications précédentes, **caractérisé en ce que** l'élément porteur (8) est réalisé sous la forme d'un circuit imprimé.

7. Système d'observation de conducteur selon l'une des revendications précédentes, **caractérisé en ce que** le projecteur d'infrarouges (10) possède au moins une diode électroluminescente à infrarouges.
